# EUROPEAN PATENT APPLICATION

(11) **EP 4 446 411 A1**
(43) Date of publication of application: **16.10.2024**
(21) Application number: 23305568.0
(22) Date of filing: 14.04.2023
(51) Int. Cl.: C12N 9/54, C11D 3/386

(54) **VARIANT SUBTILISIN PROTEASES**

(71) Applicant: PEACCEL, 97490 Sainte Clotilde (FR)
(72) Inventor: FONTAINE, Nicolas, 97419 LA POSSESSION (FR)
(74) Representative: Lavoix

(57) **Abstract**

The present invention concerns variant subtilisin proteases having improved thermostability and/or kinetic properties compared to wild-type subtilisin. These variant subtilisin proteases have advantageous properties for industrial use.

## Description

The present invention concerns variant subtilisin proteases having improved thermostability and/or kinetic properties compared to wild-type subtilisin. These variant subtilisin proteases have advantageous properties for industrial use.

Proteases are important industrial enzymes which can be applied for numerous applications ranging from leather and textile treatment to detergent additives and therapeutics. Even though there are many different forms and functions, they share the same mechanism of peptide bond scission i.e. polarization of the amide CO bond as well as activation of a nucleophilic group to attack the carbonyl carbon resulting in its hydrolysis.

One of the most prominent proteases that is used for leather and textile industry and is added to detergent is the protease subtilisin. Subtilisins have been added for example to laundry detergent since more than 40 years to facilitate the removal of different stains. Subtilisin are alkaline proteases that have a broad specificity for proteinaceous stains that commonly soil clothing, including proteins found in blood, grass, soil and many food products. Subtilisins are used in all types of laundry detergents (liquid, powder, concentrated, etc.) and in automatic dishwashing detergents.

Subtilisin further finds applicability of in various other industries including food processing and packaging, synthesis of inhibitory peptides, therapeutic, and waste management.

Initially isolated from the bacteria *Bacillus subtilis,* subtilisin has become one of the most intensively studied and extensively engineered proteins known to date (Bryan, P. N. (2000). Protein engineering of subtilisin. Biochimica et Biophysica Acta (BBA)-Protein Structure and Molecular Enzymology, 1543(2), 203-222., Maurer, K. H. (2004). Detergent proteases. Current opinion in Biotechnology, 15(4), 330-334.). A wide variety of subtilisins have been identified, and the amino acid sequences of a number of these subtilisins have been determined. Structural investigations, including more than 200 crystal structures, have revealed that subtilisins share a common active site with other serine proteases, the Ser-His-Asp catalytic triad.

Despite many structural studies, the correlation of specific functional properties (temperature, pH stability, wash performance for different stains, etc.) and the structural elements of the protein remain to be elucidated. Indeed, due both to the lack of structural predictability and to the need to optimize multiple characteristics simultaneously, the task of protein engineering remains difficult. For example, in detergent applications, subtilisins have not only to be active for a variety strains, they also have to be stable in the presence of other detergent components and additives which include, among other ingredients, other enzymes such as cellulases, lipases and bleaching agents. The subtilisin selected for such an application should also be active under a variety of specific conditions such as high or low temperature, varying pH. Finally, subtilisins should be stable in the presence of effective concentrations of other enzymes, and at the same time must not result in the degradation (proteolysis) of these enzymes. There are >200 subtilisins known and reported. Although they contain conserved amino acids such as the catalytic triad their primary sequences are highly divergent. However, their structure is highly conserved. All subtilisins have in common that they are Ca-dependent serine proteases. The size of subtilisins ranges from 18 to 90 kDa. However, most of the commercial subtilisins have a size of about 27kDa. They comprise a pro-domain, the active site, a substrate binding site and a Ca-binding site. The structure of the protein's active site includes the catalytic triad, which consists of the serine at position 221, the histidine at position 64 and the aspartic acid at position 32. The pro-domain plays a role for the correct folding and localization of the enzyme and is partly cleaved off to obtain the mature protease.

As amino acid exchanges that confer improvements are difficult to predict, and therefore difficult to engineer, subtilisin proteases have been intensively engineered by numerous directed evolution approaches such as random mutagenesis as well as rational design to tailor their properties towards industrial demands (patent applications EP0723579, EP3486319, EP3327122). Advances in subtilisin engineering comprise simultaneous improvement of thermal resistance and activity at low temperatures, different strategies to modulate pH profiles and the improvement of its wash performance. In addition, the development of new expression systems allowed decreasing production costs. Although directed evolution and rational design resulted in substantially improved subtilisins, the need to develop better enzymes remains imperative.

### Description of the invention

The inventor has developed new variants of subtilisin protease of *Bacillus lentus* that have improved thermostability and/or kinetic properties as compared with the wild-type protease.

The invention thus relates to a variant subtilisin protease that has improved thermostability and/or kcat/Km ratio compared to wild-type subtilisin of *Bacillus lentus* of sequence SEQ ID NO: 2, which comprises or consists of an amino acid sequence that differs from said wild-type subtilisin of *Bacillus lentus* by at least amino acid substitutions at the following positions:
a) 74, 99, 102, 126, 198, 212, and 254;
b) 74, 99, 102, 126, 191, 198, 212, and 254; or
c) 74, 97, 126, 204, 254, and 259.

In some embodiments, the variant subtilisin protease has improved thermostability compared to wild-type subtilisin of *Bacillus lentus* of sequence SEQ ID NO: 2. In some embodiments, the variant subtilisin protease has improved thermostability and kcat/Km ratio compared to wild-type subtilisin of *Bacillus lentus* of sequence SEQ ID NO: 2. In particular, improved thermostability is improved thermostability at 60°C, for instance at pH 8.6 or pH 10.0. In particular, the improved kcat/Km ratio is improved kcat/Km ratio at pH 8.6 or pH 10.0. In some embodiments, the thermostability or kcat/Km ratio is improved at least 1, 2, 3, 4, 5, 6, 7, 8, 9, 10, 15, 20, 25, 30, 25, 40, 45, 50, 55, 60, 65, 70, 75, 80, 85, 90, 95 or 100-fold compared to wild-type subtilisin of *Bacillus lentus* of sequence SEQ ID NO: 2.

Two or more of these variant subtilisin proteases can be combined in order to provide a mixture of variant subtilisin proteases having both improved thermostability and kcat/Km ratio.

The variant subtilisin protease encompass the substitution of any of the nineteen naturally occurring L-amino acids at the designated amino acid residue positions.

In some embodiments, the variant subtilisin protease comprises or consists of an amino acid sequence that differs from said wild-type subtilisin of *Bacillus lentus* of sequence SEQ ID NO: 2 by at least the following amino acid substitutions:
a) N74D/S99G/V102W/S126G/N198A/N212S/T254P;
b) N74D/S99G/V102W/S126G/D191E/N198G/N212S/T254P; or
c) N74D/S97D/S126G/P204I/T254P/S259N.

In some embodiments, the variant subtilisin protease that has improved thermostability and/or kcat/Km ratio comprises or consists of an amino acid sequence that differs from said wild-type subtilisin of *Bacillus lentus* of sequence SEQ ID NO: 2 by one or more additional mutation(s) (i.e. deletion, insertion or substitution of amino acid).

In other embodiments, the variant subtilisin protease comprises or consists of an amino acid sequence that only differs from said wild-type subtilisin of *Bacillus lentus* of sequence SEQ ID NO: 2 by the amino acid substitutions at the following positions:
a) 74, 99, 102, 126, 198, 212, and 254;
b) 74, 99, 102, 126, 191, 198, 212, and 254; or
c) 74, 97, 126, 204, 254, and 259.

In some embodiments, the variant subtilisin protease comprises or consists of sequence SEQ ID NO: 4, SEQ ID NO: 5 or SEQ ID NO: 6.

The invention further relates to a nucleic acid comprising a polynucleotide sequence, DNA or mRNA, in particular DNA, encoding the variant subtilisin protease according to the invention. An expression vector comprising said nucleic acid operably linked to a suitable control sequence capable of effecting the expression of said nucleic acid in a suitable host is also disclosed.

A nucleic acid encoding the variant subtilisin comprising or consisting of SEQ ID NO: 4 comprises for instance SEQ ID NO: 7. A nucleic acid encoding the variant subtilisin comprising or consisting of SEQ ID NO: 5 comprises for instance SEQ ID NO: 8. A nucleic acid encoding the variant subtilisin comprising or consisting of SEQ ID NO: 6 comprises for instance SEQ ID NO: 9.

The variant subtilisin protease may be produced recombinantly by expression of said nucleic acid in a suitable host cell, such as procaryotic or eucaryotic host, in particular a mammalian cell, an insect cell or a bacterium. The invention thus also relates to a host cell comprising (or transformed with) the nucleic acid or expression vector of the invention.

Further provided is a method of producing the variant subtilisin protease, which comprises culturing the host cell according to the invention under conditions and for a time sufficient to express the variant subtilisin protease. The method may further comprise a step of collecting and purifying the expressed variant subtilisin protease.

The variant subtilisin protease may also be produced recombinantly from a DNA template in a cell free expression (CFE) system, such as an *E.* coli-based CFE system. A CFE system typically comprises the necessary molecular components for translation, composed of the ribosomes and translation cofactors; an energy mix that mainly contains the four ribonucleosides and a phosphate donor for ATP regeneration, and optionally tRNAs; an amino acid mix, as reviewed in Garenne et al. Nat Rev Methods Primers 1, 49 (2021).

Accordingly, the invention further relates to a method of producing the variant subtilisin protease, which comprises contacting a nucleic acid comprising a polynucleotide sequence encoding a variant subtilisin protease according to the invention with a cell free expression system under conditions and for a time sufficient to express the variant subtilisin protease.

The methods of producing the variant subtilisin protease may further comprise a step of collecting and purifying the expressed variant subtilisin protease.

The variant subtilisin protease of the invention find use in various industries, in particular for leather and textile industry, but also in food processing and packaging, synthesis of inhibitory peptides, therapeutic, and waste management.

The improved thermostability and/or kinetic parameter (kcat/Km) render(s) the variant subtilisin protease particularly useful in laundry detergents and dishwashing detergents.

Accordingly, the invention further relates to a cleaning composition comprising:
a) a variant subtilisin protease of the invention; and
b) one or more cleaning composition materials compatible with the variant subtilisin protease.

In some embodiments, the cleaning composition comprises two or more subtilisin proteases of the invention, e.g. a subtilisin proteases comprising or consisting of SEQ ID NO:5 and a subtilisin proteases comprising or consisting of SEQ ID NO: 4 or 6.

The term "cleaning composition materials", as used herein, means any liquid, solid or gaseous material selected for the particular type of cleaning composition desired and the form of the product (e.g., liquid; granule; spray composition), which materials are also compatible with the variant subtilisin protease used in the composition. The specific selection of cleaning composition materials is readily made by considering the surface, item or fabric to be cleaned, and the desired form of the composition for the cleaning conditions during use (e.g., through the wash detergent use). The term "compatible", as used herein, means the cleaning composition materials does not reduce the proteolytic activity of the variant subtilisin protease to such an extent that the subtilisin protease is not effective as desired during normal use situations.

An effective amount of one or more variant subtilisin proteases described above are included in compositions useful for cleaning a variety of surfaces in need of proteinaceous stain removal. Such cleaning compositions include detergent compositions for cleaning hard surfaces, unlimited in form (e.g., liquid and granular); detergent compositions for cleaning fabrics, unlimited in form (e.g., granular, liquid and bar formulations); dishwashing compositions (unlimited in form); oral cleaning compositions, unlimited in form (e.g., dentifrice, toothpaste and mouthwash formulations); and denture cleaning compositions, unlimited in form (e.g., liquid, tablet). As used herein, "effective amount of variant subtilisin protease" refers to the quantity of variant subtilisin protease described hereinbefore necessary to achieve the enzymatic activity necessary in the specific cleaning composition. Such effective amounts are readily ascertained by one of ordinary skill in the art and is based on many factors, such as the particular variant subtilisin protease used, the cleaning application, the specific composition of the cleaning composition, and whether a liquid or dry (e.g., granular, bar) composition is required, and the like. Preferably, the cleaning composition is a fabric cleaning composition, or a dishwashing composition.

As used herein, "non-fabric cleaning compositions" include hard surface cleaning compositions, dishwashing compositions, oral cleaning compositions, denture cleaning compositions and personal cleansing compositions.

All parts, percentages and ratios used herein are by weight unless otherwise specified.

Preferably, the cleaning compositions of the present invention comprise from about 0.0001 % to about 10% of one or more variant subtilisin proteases, more preferably from about 0.001% to about 1%, more preferably still from about 0.001% to about 0.1%.

According to some embodiments, the cleaning composition materials are selected from the group consisting of surfactants, solvents, buffers, enzymes, soil release agents, clay soil removal agents, dispersing agents, brighteners, suds suppressors, fabric softeners, suds boosters, enzyme stabilizers, builders, bleaching agents, dyes, perfumes, and mixtures thereof.

When in liquid form, the cleaning compositions can contain water and other solvents as carriers. Low molecular weight primary or secondary alcohols exemplified by methanol, ethanol, propanol, and isopropanol are for instance suitable.

The cleaning composition typically comprises 0.1 % to 60%, preferably at least 1 % surfactant(s) by weight of the composition, such as from 1% to 30%, or from 30% to 60% (in the case of "concentrated" detergents), by weight of surfactant(s). The cleaning compositions can contain various anionic, nonionic, zwitterionic, etc., surfactants. In some embodiments, the surfactants comprise materials selected from the group consisting of alkyl benzene sulfonates, primary alkyl sulfates, secondary alkyl sulfates, alkyl alkoxy sulfates, alkyl alkoxy carboxylates, alkyl polyglycosides and their corresponding sulfated polyglycosides, alpha-sulfonated fatty acid esters, alkyl and alkyl phenol alkoxylates, betaines and sulfobetaines, amine oxides, N-methyl glucamides, nonionic primary alcohol ethoxylates, nonionic primary alcohol mixed ethoxy/propoxy, and mixtures thereof.

When the cleaning composition is a hard surface cleaning composition or a fabric cleaning composition, various builders may be incorporated at levels from about 5% to about 50% by weight. Typical builders include the 1-10 micron zeolites, polycarboxylates such as citrate and oxydisuccinates, layered silicates, phosphates, and the like. Accordingly, in some embodiments, the cleaning composition further comprises at least about 5% builder selected from the group consisting of zeolites, polycarboxylates, layered silicates, phosphates, and mixtures thereof.

The cleaning composition may comprise various additional enzymes, such as cellulases, lipases, amylases, peroxidases, and proteases, typically at levels of from about 0.001 % to about 1 % by weight. Various detersive and fabric care enzymes are well-known in the laundry detergent art.

The cleaning composition may comprise various bleaching compounds, such as the percarbonates, perborates and the like. They can be used in such cleaning compositions, typically at levels from about 1 % to about 15% by weight. If desired, such compositions can also contain bleach activators such as tetraacetyl ethylenediamine, nonanoyloxybenzene sulfonate, and the like, which are also known in the art. Usage levels typically range from about 1% to about 10% by weight.

The cleaning composition may comprise various soil release agents, especially of the anionic oligoester type, various chelating agents, especially the aminophosphonates and ethylenediaminedisuccinates, various clay soil removal agents, especially ethoxylated tetraethylene pentamine, various dispersing agents, especially polyacrylates and polyasparatates, various brighteners, especially anionic brighteners, various dye transfer inhibiting agents, such as polyvinyl pyrrolidone, various suds suppressors, especially silicones and secondary alcohols, various fabric softeners, especially smectite clays and clay floculating polymers (e.g., poly(oxy ethylene)), and the like can all be used in such compositions at levels ranging from about 1 % to about 35% by weight.

Enzyme stabilizers may also be used in the cleaning compositions of the present invention. Such enzyme stabilizers include propylene glycol (preferably from about 1% to about 10%), sodium formate (preferably from about 0.1% to about 1%) and calcium formate (preferably from about 0.1% to about 1%).

The cleaning compositions herein is preferably formulated such that during use in aqueous cleaning operations, the wash water will have a pH between about 6.8 and about 11.0. Finished products thus are typically formulated at this range. Techniques for controlling pH at recommended usage levels include the use of buffers, alkalis, acids, etc., and are well known to those skilled in the art.

The improved thermostability and/or kinetic parameter (kcat/Km) also render(s) the variant subtilisin protease useful in food processing and/or packaging, for synthesis of peptides, for therapeutic use, or for waste management.

In particular subtilisin is a protease that has a wide range of pharmaceutical applications. Some examples of these applications include:
- Production of protein therapeutics: Subtilisin is used in the production of biologic drugs such as monoclonal antibodies and recombinant proteins.
- Enzyme replacement therapy: Subtilisin can be used to produce enzymes that are deficient in patients with genetic diseases, such as cystic fibrosis and Gaucher's disease.
- Digestive enzyme supplements: Subtilisin can be used as a digestive enzyme supplement for individuals who are unable to produce enough digestive enzymes, such as those with pancreatic insufficiency.
- Wound care: Subtilisin is used in some wound care products to help remove necrotic tissue and promote healing.
- Anti-inflammatory therapy: Subtilisin has been studied as a potential anti-inflammatory agent, as it has been shown to degrade cytokines and other inflammatory molecules.

Overall, subtilisin is a versatile enzyme with many potential applications in the pharmaceutical industry.

Accordingly, the invention further relates to a composition comprising at least one variant subtilisin protease of the invention, e.g. two variant subtilisin protease(s) of the invention or more, and optional additional component(s) which nature may vary depending on the industrial use of the composition, e.g. for food processing and/or packaging, for synthesis of peptides, for therapeutic use, or for waste management.

In an embodiment, the invention provides for a pharmaceutical composition comprising at least one variant subtilisin protease of the invention, and a pharmaceutically acceptable carrier.

The invention will be further illustrated by the following examples.

### Example

### Example 1: Data extraction from patent application WO1995010591 A1 and modelling

Before measurement in wet-lab, the claimed subtilisin variants were identified by using data of patent application WO1995010591 A1 as input for the processing based on the modelling methods described in patent WO 2016/166253.

### Data extraction

Patent application WO1995010591 A1 provided data on different in vitro tests (proteolytic assay, thermal stability assay) and real wash test for subtilisin variants.

Tables III, V, VI, VII from patent application WO1995010591 A1 were used to assemble 4 input datasets for the modelling. Each dataset contains the sequences of a group of subtilisin variants and the performances of the variants for one selected enzymatic parameter. The selected enzymatic parameters are the kinetic parameters kcat and Km, the thermal stability and the wash performance.

### Modelling

The 4 datasets were used as learning dataset to generate 4 models, one by selected enzymatic parameter (kcat, Km, thermal stability). The modelling method was the protocol described in the patent application published as WO 2016/166253. For the modelling of Km dataset, -log10(Km) was used instead of Km as input in order to help the model to predict Km values always superior to 0. For the kcat modelling, several numerical encodings were employed as described in the patent WO 2020/016365. The modelling algorithm was partial least square regression, pls. The numbers of components for the tuning of the pls algorithm and the used numerical encodings are described in Table 1.

**Table 1: numbers of components for pls algorithm and numerical encodings**

| Dataset input | Id of the numerical encoding in AAindex database* | Number of components for pls algorithm |
|---|---|---|
| kcat(s-1) | CHAM830104, CEDJ970102 | 7 |
| -log10(Km) | NAKH900102 | 9 |
| Thermal stability (% half-life) | WILM950101 | 22 |

| | | |
|---|---|---|
| *: AAindex is a database of numerical indices representing various physicochemical and biochemical properties of amino acids and pairs of amino acids and is available at https://www.genome.jp/aaindex /. All data are derived from published literature. CHAM830104: The number of atoms in the side chain labelled 2+1 (Charton, M. and Charton, B.T J J. Theor. Biol. 111, 447-450 (1983) CEDJ970102 : Composition of amino acids in anchored proteins (percent) (Cedano, J., et al., Mol. Biol. 266, 594-600 (1997) NAKH900102 : SD of AA composition of total proteins (Nakashima et al., J Proteins 8, 173-178 (1990)) WILM950101 : Hydrophobicity coefficient in RP-HPLC, C18 with 0.1%TFA/MeCN/H2O (Wilce et al. J Anal Chem. 67, 1210-1219 (1995)) | | |

### Selection of new mutants

The mutations of the patent application WO1995010591 A1 were used to construct new recombinant mutants with no reported values for the 3 previous parameters in the patent application WO1995010591 A1. The size of the space of possible mutants to predict from the mutations is more than 1.02*10^11sequences. The models predicted the enzymatic parameter values of these new mutants. Mutants with at least one parameter superior to the best mutant of the patent for the targeted parameter or mutants with all the values parameters superior to the *Bacillus lentus* WT (SEQ ID NO: 10 of patent application WO1995010591 A1; SEQ ID NO: 2 herein) were filtered.

Next, some of these mutants were measured in wet-lab.

### Expression and testing of mutants

Three selected mutants as well as the wild-type and one mutant of the patent application WO1995010591 A1 data set were cloned in a *Bacillus* vector behind a constitutive APRE promotor and expressed in a protease deficient *Bacillus subtilis* 168 strain. The transformed strains were grown for 24 hours at 37°C in shake flasks. The subtilisin variants were purified by Ion exchange chromatography followed by extraction with a 0-200 mM salt gradient. The activity of the protease variants was determined by the release of the chromophore para-nitroanilide from succinyl-alanine-alanine-proline-phenylalanine-para-nitroanilide (suc-AAPFpNA). The release of the pNA causes an increase in the absorbance at 410 nm, the temporal progression of which is used as a measure of the activity of the enzymes.

### Example 2: kinetic parameters of subtilisin mutants

The kinetic parameters kcat and Km of the variants were measured using basically the method described in Bonneau, P. R., Graycar, T. P., Estell, D. A., & Jones, J. B. (1991). Alteration of the specificity of subtilisin BPN'by site-directed mutagenesis in its S1 and S1'binding sites. Journal of the American Chemical Society, 113(3), 1026-1030. A small aliquot of purified subtilisin variant was added to a 96 microtiterplate well containing succinyl-L-Ala-L-Ala-L-Pro-L-Phe-p-nitroanilide (SEQ ID NO: 1) dissolved in 0.1M Tris-HCL, pH 8.6. The reaction progress was measured spectrophotometrically at 25°C by monitoring the absorbance of p-nitroaniline at 410nm. The corresponding experimental values were fit to the Michaelis-Menten equation by a regression algorithm. The kcat/KM ratio was obtained by dividing the resulting values. Table 2 shows the results of the kinetic measurements at pH 8.6.

**Table 2: kinetic parameters kcat and Km of the variants at pH 8.6**

| | kcat pH 8.6 (% of wild-type) | Km pH 8.6 (mM) | kcat/Km pH 8.6 (% of wild-type) |
|---|---|---|---|
| WT (SEQ ID NO :2) | 100 | 0.63 | 100 |
| N74D/S101A/V102W (control variant of WO1995010591; sequence SEQ ID NO: 3) | 638 | 0.29 | 1392 |
| N74D/S99G/V102W/S126G/D191E/N198G/N212 S/T254P (variant of sequence SEQ ID NO: 4) | 579 | 0.11 | 3326 |
| N74D/S99G/V102W/S126G/N198A/N212S/T254 P (variant of sequence SEQ ID NO: 5) | 503 | 0.05 | 6362 |
| N74D/S97D/S126G/P204I/T254P/S259N (variant of sequence SEQ ID NO: 6) | 55 | 0.66 | 51 |

The kinetic parameters kcat and Km of the variants were also measured using basically the method described in Bonneau, P. R., Graycar, T. P., Estell, D. A., & Jones, J. B. (1991). Alteration of the specificity of subtilisin BPN'by site-directed mutagenesis in its S1 and S1'binding sites. Journal of the American Chemical Society, 113(3), 1026-1030. A small aliquot of purified subtilisin variant was added to a microtiterplate containing suc-AAPFpNA (SEQ ID NO:1) dissolved in 0.1M glycine pH10. The reaction progress was measured spectrophotometrically by monitoring the absorbance of p-nitroaniline at 410nm. The corresponding experimental values were fit to the Michaelis-Menten equation by a regression algorithm. The kcat/Km ratio was obtained by dividing the resulting values. Table 3 shows the results of the kinetic measurements at pH 10.0. Kcat kcat/km are indicated as % compared to the wild-type (100%).

**Table 3: kinetic parameters kcat and Km of the variants at pH 10**

| | kcat pH 10 (%half-life of wild-type) | Km pH 10 (mM) | kcat/Km pH10 (% wild-type) |
|---|---|---|---|
| WT (SEQ ID NO :2) | 100 | 1.34 | 100 |
| N74D/S101A/V102W (control variant of WO1995010591; sequence SEQ ID NO: 3) | 554 | 0.33 | 2263 |
| N74D/S99G/V102W/S126G/D191E/N198G/N212S/T254P (variant of sequence SEQ ID NO: 4) | 719 | 0.19 | 5100 |
| N74D/S99G/V102W/S126G/N198A/N212S/T254P (variant of sequence SEQ ID NO: 5) | 723 | 0.09 | 10827 |
| N74D/S97D/S126G/P204I/T254P/S259N (variant of sequence SEQ ID NO: 6) | 37 | 0.52 | 97 |

### Example 3: Thermostability of subtilisin mutants

Thermostability measurements were carried out at 60°C pH 8.6. Purified enzyme was added to a tube containing 0.1M Tris HCl pH 8.6 thermostated at 60°C final concentration of 1nM (PS4, PS10) or 50nM (wild-type, PS18) enzyme.

Aliquots were removed from the 60°C incubation at various times and immediately assayed for enzyme activity by addition to a microtiterplate containing 10mM of the synthetic peptide substrate succinyl-L-Ala-L-Ala-L-Pro-L-Phe-p-nitroanilide (SEQ ID NO:1) dissolved in 0.1M Tris-HCl buffer pH 8.6 thermostated at 25°C. The absorbance of the reaction product p-nitroaniline at 410nm was measured after 5 minutes. The half-life was determined as a function of the time of the incubation at 60°C. Half-life was defined as time required for 50% enzyme inactivation. The results are presented in Table 4 as percent of half-life of the wild-type *Bacillus lentus* subtilisin (GG36).

**Table 4: relative half-life of subtilisin variants at pH 8.6, 60°C**

| Variant | % pH 8.6 |
|---|---|
| WT (SEQ ID NO:2) | 100 |
| N74D/S101A/V102W (control variant of WO1995010591; sequence SEQ ID NO: 3) | 400 |
| N74D/S99G/V102W/S126G/D191E/N198G/N212S/T254P (variant of sequence SEQ ID NO: 4) | 800 |
| N74D/S99G/V102W/S126G/N198A/N212S/T254P (variant of sequence SEQ ID NO: 5) | 566.67 |
| N74D/S97D/S126G/P204I/T254P/S259N (variant of sequence SEQ ID NO: 6) | 1 183.33 |

Thermostability measurements were also carried out at pH 10 and 60°C temperature. To a tube containing 0.1M Glycine pH 10.0 thermostated at 60°C purified enzyme was added to give a final concentration of 1nM (PS4, PS10) or 50nM (wild-type, PS18) enzyme.

Aliquots were removed from the 60°C incubation at various times and immediately assayed for enzyme activity by addition to a microtiterplate containing 10mM of the synthetic peptide substrate succinyl-L-Ala-L-Ala-L-Pro-L-Phe-p-nitroanilide (SEQ ID NO:1) dissolved in 0.1M Tris-HCl buffer pH 8.6 thermostated at 25°C. The absorbance of the reaction product p-nitroaniline at 410nm was measured after 5 minutes. The half-life was determined as a function of the time of the incubation at 60°C. Half-life was defined as time required for 50% enzyme inactivation. The results are presented as percent of half-life of the wild-type *Bacillus lentus* subtilisin (GG36).

**Table 5: relative half-life of subtilisin variants at pH 10, 60°C**

| Variant | Ratio pH 10 |
|---|---|
| WT (SEQ ID NO:2) | 100 |
| N74D/S101A/V102W (control variant of WO1995010591; sequence SEQ ID NO: 3) | 250 |
| N74D/S99G/V102W/S126G/D191E/N198G/N212S/T254P (variant of sequence SEQ ID NO: 4) | 266.67 |
| N74D/S99G/V102W/S126G/N198A/N212S/T254P (variant of sequence SEQ ID NO: 5) | 1 066.67 |
| N74D/S97D/S126G/P204I/T254P/S259N (variant of sequence SEQ ID NO: 6) | 1 266.67 |

## Claims

1. A variant subtilisin protease that has improved thermostability and/or kcat/Km ratio compared to wild-type subtilisin of *Bacillus lentus* of sequence SEQ ID NO: 2, which comprises an amino acid sequence that differs from said wild-type subtilisin of *Bacillus lentus* by at least amino acid substitutions at the following positions:
a) 74, 99, 102, 126, 198, 212, and 254;
b) 74, 99, 102, 126, 191, 198, 212, and 254; or
c) 74, 97, 126, 204, 254, and 259.

2. The variant subtilisin protease according to claim 1, which comprises an amino acid sequence that differs from wild-type subtilisin of *Bacillus lentus* of sequence SEQ ID NO: 2 by at least the following amino acid substitutions:
a) N74D/S99G/V102W/S126G/N198A/N212S/T254P;
b) N74D/S99G/V102W/S126G/D191E/N198G/N212S/T254P; or
c) N74D/S97D/S126G/P204I/T254P/S259N.

3. The variant subtilisin protease according to claim 1 or 2, which comprises sequence SEQ ID NO: 4, SEQ ID NO: 5 or SEQ ID NO: 6.

4. The variant subtilisin protease according to any one of claims 1 to 3, which has improved thermostability at 60°C over said wild-type subtilisin of *Bacillus lentus.*

5. A nucleic acid comprising a polynucleotide sequence encoding the variant subtilisin protease according to any one of claims 1 to 4.

6. A host cell comprising the nucleic acid according to claim 5.

7. A method of producing the variant subtilisin protease according to any one of claims 1 to 4, which comprises culturing the host cell according to claim 6 under conditions and for a time sufficient to express the variant subtilisin protease, or contacting a nucleic acid according to claim 5 with a cell free expression system under conditions and for a time sufficient to express the variant subtilisin protease.

8. A cleaning composition comprising:
a) a variant subtilisin protease as defined in any one of claims 1 to 4; and
b) one or more cleaning composition materials compatible with the variant subtilisin protease.

9. The cleaning composition according to claim 8, wherein the cleaning composition materials are selected from the group consisting of surfactants, solvents, buffers, enzymes, soil release agents, clay soil removal agents, dispersing agents, brighteners, suds suppressors, fabric softeners, suds boosters, enzyme stabilizers, builders, bleaching agents, dyes, perfumes, and mixtures thereof.

10. The cleaning composition according to claim 8 or 9, wherein the cleaning composition materials comprise at least 1 % surfactant by weight of the composition, said surfactant comprising materials selected from the group consisting of alkyl benzene sulfonates, primary alkyl sulfates, secondary alkyl sulfates, alkyl alkoxy sulfates, alkyl alkoxy carboxylates, alkyl polyglycosides and their corresponding sulfated polyglycosides, alpha-sulfonated fatty acid esters, alkyl and alkyl phenol alkoxylates, betaines and sulfobetaines, amine oxides, N-methyl glucamides, nonionic primary alcohol ethoxylates, nonionic primary alcohol mixed ethoxy/propoxy, and mixtures thereof.

11. The cleaning composition according to any one of claims 8 to 10, further comprising at least about 5% builder selected from the group consisting of zeolites, polycarboxylates, layered silicates, phosphates, and mixtures thereof.

12. The cleaning composition according to any one of claims 8 to 11, comprising:
(a) from about 0.0001 % to about 10% a variant subtilisin protease as defined in any one of claims 1 to 4;
(b) at least about 5% surfactant;
(c) at least about 5% builder; and
(d) optionally, one or more cleaning composition materials compatible with the protease enzyme selected from the group consisting of solvents, buffers, enzymes, soil release agents, clay soil removal agents, dispersing agents, brighteners, suds suppressors, fabric softeners, suds boosters, enzyme stabilizers, bleaching agents, dyes, perfumes, and mixtures thereof.

13. The cleaning composition according to any one of claims 8 to 12, which is a fabric cleaning composition, or a dishwashing composition.

14. A composition comprising at least one variant subtilisin protease as defined in any one of claims 1 to 4.
